# EUROPEAN PATENT SPECIFICATION

(11) **EP 4 407 336 B1**
(45) Date of publication and mention of the grant of the patent: **14.10.2026**
(21) Application number: 23153006.4
(22) Date of filing: 24.01.2023
(51) Int. Cl.: G01R 33/56, G01R 33/28

(54) **DEVICE AND METHOD FOR 3D REAL-TIME MEDICAL INSTRUMENT TRACKING**
VORRICHTUNG UND VERFAHREN ZUR VERFOLGUNG MEDIZINISCHER 3D-INSTRUMENTE IN ECHTZEIT
DISPOSITIF ET PROCÉDÉ DE SUIVI D'INSTRUMENT MÉDICAL EN TEMPS RÉEL 3D

(43) Date of publication of application: 31.07.2024
(73) Proprietor: Siemens Healthineers AG, 91301 Forchheim (DE); King's College London, London WC2R 2LS (GB)
(72) Inventor: Grzegorz, Kowalik, London, N22 5HP (GB); Roujol, Sebastien, Kingston upon Thames, KT2 6JB (GB); Neji, Radhouene, London, N65XP (GB); Pushparajah, Kuberan, London, Se23 3XN (GB); Razavi, Reza, Northwood, HA6 3ES (GB)
(74) Representative: Siemens Healthineers Patent Attorneys

(56) References cited:
- WO-A2-02/46784
- US-A1- 2007 043 288
- US-A1- 2007 249 934
- US-A1- 2012 089 008

## Description

The invention is related to a method and a magnetic resonance tomography unit for tracking a medical instrument. A tracking volume is defined enclosing a predefined trajectory of the medical instrument. Images of the tracking volume in presence of the medical instrument are acquired with the magnetic resonance tomography unit and coordinates of the medical instrument are extracted and output on a display.

Magnetic computer tomographs are imaging facilities, which for imaging an examination object align nuclear spins of the examination object with a strong external magnetic field and excite them with a magnetic alternating field for precession about this alignment. The precession or return of the spins from this excited state back into a state with lower energy in its turn creates as a response a magnetic alternating field, which is received via antennas.

A spatial encoding is impressed on the signals with the aid of magnetic gradient fields, which subsequently makes it possible to assign the received signal to a volume element. The received signal is then evaluated and a 3D image representation of the examination object is provided.

Such full 3D image acquisition requires some time to reach a sufficient image quality, in particular signal to noise ratio. Furthermore, most medical instruments made from metal or plastics cannot be imaged directly by the magnetic resonance tomography, as the material of the instrument does either provide no magneto-resonance active spins or at least not at a frequency like the Larmor frequency usually used for imaging the water in the body of a patient. Unless the medial instruments are provided with magnet-resonance active materials or devices, which add to the dimensions of the device or are costly, the imaging of the medical instrument relies on indirect effects like absence of water in the device or artefacts generated by the conductivity or different susceptibility of the material of the medical device, which makes imaging even more difficult. When real time tracking of a device is required, the short integration times do not allow full 3D imaging at a sufficient image quality.

Document US 2007/0043288 A1 discloses a tracking device for tracking a tip of an interventional instrument during an interventional procedure performed on an associated subject and monitored by magnetic resonance imaging. The device includes a resonant circuit disposed at the tip of the catheter. The resonant circuit includes a coil having a coil inductance and a light-sensitive metal-insulator-semiconductor capacitor optically coupled with an optical fiber and having a selected capacitance determined by an intensity of light delivered by the optical fiber. A selected resonance frequency of the resonant circuit is determined by the coil inductance and the selected capacitance. The resonance frequency is adjusted by modulating the intensity of light delivered to the light-sensitive metal-insulator-semiconductor capacitor.

Document US 2007/0249934 A1 discloses an MR system and method for tracking a device of an interventional procedure within a scan subject. At least two MR projections of the device are acquired, from which 3D coordinates of the device are determined. Subsequent image acquisition is adjusted with respect to the coordinates of the device to guide movement thereof towards target anatomy.

Document US 2012/0089008 A1 discloses a method for tracking a needle along a path under real-time magnetic resonance imaging (MRI) guidance. The method includes extracting a candidate needle artifact region from a plurality of incoming MR images of a needle inside biological tissue, determining an initial needle direction vector from the candidate needle artifact region in each of the plurality of incoming needle images, detem1ining an initial needle base point from an intersection of the needle direction vector with a first incoming needle image, and an initial needle tip point from an intersection of the needle direction vector with a last incoming needle image, and tracking said needle by minimizing a cost function of the initial base point and initial direction vector to calculate an updated base point and updated direction vector. The updated base point and direction vector are used to visualize the advance of the needle along the path through the biological tissue.

It is therefore an object of the invention to improve the tracking of a medical instrument.

The object is achieved by an inventive magnetic resonance tomography unit for tracking a medical instrument as claimed in claim 1, a method for tracking a medical instrument with a magnetic resonance tomography unit as claimed in claim 10, and a corresponding computer program product as claimed in claim 13

The method according to the invention is a method for tracking a medical instrument with a magnetic resonance tomograph in real time. Real time means in his context that the image repetition rate is high enough such that an operator can track and control the movement of the medical instrument, e.g. with an image repetition rate higher than 1 f/s (frames per second), 5 f/s or 10 f/s.

The method is executed by a magnetic resonance tomograph which comprises a control unit for controlling all sub-systems like gradient generation and RF transmission and reception for image acquisition and also for the image reconstruction, analysis and display. The control unit may also be separated in different sub-systems for each task.

In one step of the method, a tracking volume is defined or determined by the control unit. A tracking volume is defined as a volume in the field of view of the magnetic resonance tomograph which encloses a predefined trajectory of the medical instrument. The predefined trajectory may be entered by an operator or transferred as data from a treatment planning system. The trajectory describes a path along which the medical instrument shall be moved. The tracking volume may e.g. be determined by a cuboid wherein the surfaces are determined by six planes orthogonal to the axes of a coordinate system in which the trajectory is defined. The coordinates where the planes intersect with the axes are the minimum and the maximum value of the coordinate of the trajectory along each axe. Other non-orthogonal coordinate systems and tracking volumes may also be considered, wherein the trajectory is included by the tracking volume. The tracking volume may also be changed during execution of the method, which might be considered as starting with a new instance of the method.

In another step of the method, a first 2D projection image of the tracking volume is acquired by the control unit in presence of the medical instrument with the magnetic resonance tomography unit. The tracking volume is, as explained before, a three-dimensional volume which extends in all three directions of space. In particular, for the object to be imaged with a resolution of r, i.e. the volume elements to be image being cubes with a side length r, the tracking volume has an extension in each dimension of a multiple of r. A 2D projection image of the tracking volume is a 2D image, i.e. an array of pixels arranged in a matrix in a 2D surface, preferably a plane or a piece of a plane. The plane could be considered as a section plane through the tracking volume.

Each pixel is assigned a value. In a 2D projection image, this value is not assigned to the magnetic resonance signal of a single volume element or voxel in the tracking volume, but derives from a plurality of voxels arranged along a line through the pixel in a projection direction. Usually, the projection direction is perpendicular to the 2D surface. Typically, the values of each voxel along the line sum up to a value which is assigned to the pixel. This is similar to a 2D X-ray image, wherein the attenuation factors of all volume elements along the x-ray beam direction sum up to the pixel value in the x-ray image.

The control unit determines the first reference 2D image from a plurality of first 2D projection images and/or determines the second reference 2D image from a plurality of second 2D projection images. As discussed before, the reference 2D images shall depict features in the tracking volume, which do not change with the movement of the medical instrument and may be subtracted as background from the 2D projection images to ease detection of the medical instruments. Such reference 2D images may be derived from a plurality of 2D projection images by selecting or filtering constant image content. As a most simple way, a plurality of 2D projection images could be averaged, such that constant content adds up while changing content is reduced. Median functions could be applied, which identify and suppress pixels changing in consequent 2d projections. The determination may also depend on the signal generated by the medical device. E.g. a metallic instrument causes extinction of the MR signal in a surrounding volume, while a catheter filled with contrast agent like a Gadolinium solution may cause a strong positive signal.

In a beneficial way, generating the reference 2D images from 2D projection images allow a continuous update of the background signal and saves time for separate acquisition of reference 2D images.

The contrast of the image acquisition of the first 2D projection image may be enhanced by contrast improving steps like partial saturation pre-pulses.

In a similar way, in another step a second 2D projection image is acquired. But a first projection direction or projection vector for the first 2D projection image is different from a second projection vector of the second 2D projection image. The first projection vector and the second projection vector enclose an angle larger than 30 degrees or 60 degrees, preferably equal to 90 degrees, such that the 2D projection images may be used as 2D maps showing the tracking volume from different viewing directions, as will be discussed later. Preferably the projection vectors are orthogonal to planes of the 2D projection images.

In another step of the method, a first reference 2D image is determined by the control unit. A reference 2D image is an image similar to the first and second 2D projection image. It shows the tracking volume under the same viewing direction or projection direction. In particular, a reference 2D image depicts features of the patient in the tracking image, which do not change with the movement of the medical instrument. As discussed beyond, such a reference 2D image can be derived from a separate image acquisition in absence of the medical instrument. Or a reference 2D image may be computed by the control unit from previous first and second 2D images, eliminating the variable effects caused by the moving medical instrument.

In another step of the method, in a similar way, a second reference 2D image is determined by the control unit, having the same viewing direction or projection direction as the second 2D projection image.

In another step of the method, a background is suppressed or removed from the first 2D image as a function of the first reference 2D image by the control unit. As the first 2D reference image and the first 2D image basically represent the same volume from the same perspective, the background may be suppressed by a subtraction of the pixel values of the first 2D image minus the first 2D reference image. As the images may differ slightly by contrast or an offset, some corrections may be required to achieve best results. The pixel values may e.g. be scaled by a linear function y = ax+b before subtracting, wherein y is the pixel value for the first 2D image and x the pixel value for the first 2D reference image. The values for the scaling function may be determined by an optimization problem, e.g. minimizing the sum of the square values of the difference of the image pixel values (LSQR).

In another step of the method, the control unit determines a first set of coordinates of the medical instrument from the first 2D image after background suppression. In this image, major contrast shall be caused by the medical device. E.g. an algorithm for detecting areas of high contrast may therefore be applied to determine the contour of the medical instrument. Other image analysis algorithms may be used, as well as neuronal networks and/or AI trained with images of the medical instrument at known coordinates. With the coordinates of the contour in the image, coordinates of the medical instrument in the plane of the image may be determined using the mapping relation of the imaging process of the magnetic resonance tomograph.

In a similar way, in another two steps of the method
a background is suppressed or removed from the second 2D image as a function of the second reference 2D image by the control unit and a second set of coordinates in the image plane of the second 2D reference image or the second 2D image of the medical instrument is determined from the second 2D image by the control unit.

In other words, at least two set of coordinates in two planes at different orientations in space are known, which define a position of the medical instrument, i.e. at least one point like the tip, in three dimensional space.

In another step of the method, the 3D coordinate is extracted from the two sets of coordinates in the two planes. As the planes have different and known orientation in space, the two coordinate sets in the two planes define a position in 3D space, which is derived by coordinate transformation by the control unit.

In an advantageous manner the method allows a quick detection and 3D tracking of the medical instrument with the magnetic resonance tomograph.

The magnetic resonance tomograph configured to execute the method shares the benefits of the method according to the invention.

In an embodiment of the method according to the invention, the method further comprises the step of outputting the 3D position of the medical instrument to a user. The 3D position may e.g. be depicted in a 3D- rendered image, a virtual reality device or by a plurality of 2D images from different view aspects. It is also conceivable that the 3D position is output in numerical values or coordinates.

The visual display of the coordinates eases in a beneficial way the navigation of the medical instrument by a user.

In an embodiment of the method according to the invention, the method further comprises the step of acquiring a 2D slice comprising the medical instrument by the magnetic resonance tomograph. Within this step, the control unit determines the position of the 2D slice in dependence of the first set of coordinates and/or second set of coordinates. The control unit may e.g. determine a 3D coordinate in space as explained before and then determine a plane which includes the 3D coordinates of the medical instrument, which is given by e.g. the coordinate of the tip of the medical instrument and the direction of extension. The slice to be imaged is the cross section of this plane and the field of view.

In a beneficial way, the acquisition of the slice allows to show the user the medical instrument together with the environment in the body of a patient.

In another embodiment of the method the control unit determines the first reference 2D image by acquiring a third 2D projection image of the tracking volume in absence of the medical instrument in the tracking volume with the magnetic resonance tomography unit and/or determines the second reference 2D image by acquiring a fourth 2D projection image of the tracking volume in absence of the medical instrument in the tracking volume with the magnetic resonance tomography unit. In other words, the reference 2D images are acquired by the control units with separate image acquisitions of 2D projection images. These reference 2D images are acquired when the medical instrument is not present in the tracking volume, such that no signals caused by the medical instrument are part of the reference 2D images. The reference 2D images may e.g. be acquired in advance to the intervention. The acquisition may be performed using a fat suppressing sequence e.g. a Dixon sequence or a sequence using a fat saturating pre-pulse or a water selective exciting pulse. Also all other image improving steps explained before may be applied.

In a beneficial way the acquisition of reference 2D images without presence of the medical instrument eases detection of the medical device in subsequent images.

In an embodiment of the method, which is outside the scope of the invention as defined by the claims, the steps of acquiring a 2D projection image of the tracking volume comprises the step of exciting the spins in the tracking volume by an excitation pulse at the same time with the same excitation pulse. This might be caused with a narrow-band excitation pulse at Larmor frequency in a homogeneous B0 magnetic field. It is also conceivable to use a broadband excitation pulse as a magnetic gradient field is applied at the same time. The bandwidth of the pulse is determined by the strength of the gradient and the dimension of the tracking volume along the axis of the gradient. This selection of a single slice limited to the tracking volume suppresses signals from outside the tracking volume at least in direction of the gradient axis.

To provide a 2D projection image, during readout only a 2D encoding scheme, e.g. phase encoding, is applied in the sequence. By applying only encoding in two dimensions, e.g. x- and y-axis, there is no differentiation for the MR signals of the volume elements along the third axis and the MR signals of the volume elements are summed up.

In a beneficial way, with a 2D encoding, the complete tracking volume can be acquired in a single acquisition in a 2D projection image.

In the inventive method, in a step the defined tracking volume is divided by the control unit into a stack of slices. For the cuboid described before, such slices could e.g. provided by splitting the cuboid by a plurality of planes parallel to one of the surfaces of the cuboid. The planes could be preferably positioned equidistant, i.e. each slice having a same thickness or dimension along the normal vector of the planes. As the geometry of imaging with the magnetic resonance tomograph is distorted by field inhomogeneities the slices may also be maps of the ideal planar planes described before, distorted by the magnetic resonance tomographs imperfection. Preferably, the slices are arranged along an axe of the image acquisition, such that in the hereinafter described step of projection maybe performed as easy as possible.

In the inventive method, the magnet resonance tomograph acquires a first 1D projection for each slice. The spins to be detected in the slices are excited by an excitation pulse with a magnetic gradient applied in orthogonal direction to the slice. A 1D projection is an image acquisition, wherein an encoding for spatial resolution is only applied in one dimension. In other words, for a two-dimensional slice, i.e. a slice which has in direction of thickness only a single layer of volume elements to be acquired/imaged, an encoding is provided only along one axis. As a result, the signal generated and acquired for any spot along this axis represents an integral of the signals of all volume elements of the slice along a projection line through the axis at this spot and perpendicular to the axis in the plane of the slice. The value generated for each spot represents the integral of the spins along the projection line, similar to the electron density for of an x-ray image.

In the inventive method, the generated 1D projections for all slices are combined to a 2D projection image by the control unit. Like the lines of a TV image, the lines of the 1D projections are aligned in parallel, thereby providing an image of the stack of slices in a perspective parallel to the slices and perpendicular to the axis.

According to the invention, in another step, a second 1D projection for each slice is acquired by the magnetic resonance tomograph in a second projection direction, wherein the first projection direction and the second projection direction enclose an angle larger than 45 degrees, 60 degrees or 80 degrees, preferably of 90 degrees.

In another step of the inventive method and in a similar way as described before, the stack of second 1D projections is combined by the control unit to a second 2D projection image. The perspective of the second reference image is turned relative to the first reference image around the axis by the angle, preferably by 90 degrees to provide a view from two different sides of a cube which eases the navigation of the medical instrument by a user.

In a beneficial way, the acquisition of single slices makes it possible to cover also tracking volumes with large dimensions.

In an embodiment of the method, the method further comprising the step of acquiring an MR image for a 2D slice comprising the medical instrument, wherein the control unit determines the position of the 2D slice in dependence of the first set of coordinates and/or second set of coordinates. As the position of the medical instrument has been determined, the control unit calculates a plane with position and orientation, such that the medical instrument lies in the plane/slice. The control unit acquires then a 2D image of the slice with the magnetic resonance unit and preferably presents the image to an operator.

In a beneficial way, the method allows to provide 2D images with the medical instrument updated with a high frequency.

In an embodiment of the method according to the invention, the steps of determining reference 2D images are repeated for different positions of a patient. The position is not limited to the externally visible position of the patient, but particularly comprises a position of organs within the tracking volume. The position of organs may change during breathing and/or heartbeat. The different positions may include different phases of heartbeat or breathing. A plurality of first reference 2D images and second reference 2D images are generated by the control unit. As the 2D projection images are acquired at different positions, each of the plurality of the first reference images and of the second reference images represent a different breathing position. In other words, a library of reference images is prepared for a plurality, or preferably for all breathing and/or heartbeat positions.

In a beneficial way, background information is provided for different breathing positions which enables tracking at any time.

In an embodiment of the method according to the invention, during the steps of removing the background the control unit selects the first reference 2D image of the plurality of first reference 2D images, which is most similar to the first 2D projection image. Most similar means that the difference between the reference image and a target image with the medical instrument for tracking is least. For example, the reference image from the plurality of reference 2D images may be chosen, for which a sum of the squares of the difference of the pixel values between reference image and the target images is minimal. Most similar can also mean that a correlation function provides the highest value for the two images. There may also be some preprocessing, like displacement of the images, rotation, scaling or adjustment of lightness and/or contrast.

In a beneficial way, the peak detection
selection of the reference with the least difference eases detection of the medical instrument.

In an embodiment of the method according to the invention, in steps of acquiring a first 2D projection image and/or acquiring a second 2D projection image a fat suppressing sequence is used for acquisition. Such sequence may e.g. be a Dixon sequence or a sequence using a fat saturating pre-pulse or a water selective exciting pulse.

In a 2D projection image acquisition, fat accumulations along the projection path may dominate result of the projection. In a beneficial way, therefore a fat suppression technology may be used to reduce the fat signal and make the medical instrument visible in the projection.

In an embodiment of the method according to the invention, in the steps determining a first set of coordinates of the medical instrument from the first 2D image and /or determining a second set of coordinates of the medical instrument from the second 2D image a peak detection algorithm is applied. Such peak detection algorithm may use algorithms for e.g. a simple high-pass filter for increasing the contrast of a border, border detection or even a neuronal network trained with different images of the medical instrument at different positions. Parametrized models of the medical instruments may also be used in an optimization with LSR to determine the position of the medical instrument. Preferably the peak detection is applied to a 2D projection image after removing the background.

In a beneficial way, the peak detection algorithms speed up and make the detection of the medical instrument more reliable.

In an embodiment of the method according to the invention, the medical instrument is a catheter. A catheter may differ in the way it is imaged by the magnetic resonance tomography unit. Instead of extinction of the signal by metal, a contrast agent in the catheter may provide a strong signal. In a beneficial way, the method is independent on the kind of signal.

The tomography unit according to the invention is configured to perform the method according to the invention. The magnetic resonance tomography unit may be enabled to perform the method by loading a computer program product from a storage media into a memory of the control unit and executing the program.

The characteristics, features and advantages of this invention described above and also the manner in which these are achieved will become clearer and easier to understand in conjunction with the description given below of the exemplary embodiments, which are explained in greater detail in conjunction with the drawings.

In the figures:
- Fig. 1: shows an example of a schematic diagram of a magnetic resonance tomography unit according to the invention;
- Fig. 2: shows an example of a flow chart of the method according to the invention, steps S25, S26, and S35, S36 do not form part of the invention, but are part of an example that lies outside the scope of the invention;
- Fig. 3: shows a schematic view of a tracking volume with a trajectory;
- Fig. 4: shows a schematic view of a 1D projection of a slice;
- Fig. 5: shows a schematic view of a projection of the trajectory onto a x-z-plane;
- Fig. 6: shows a schematic view of a projection of the trajectory onto a y-z-plane;
- Fig. 7: shows a schematic view of a 2D image projection using 2D encoding.

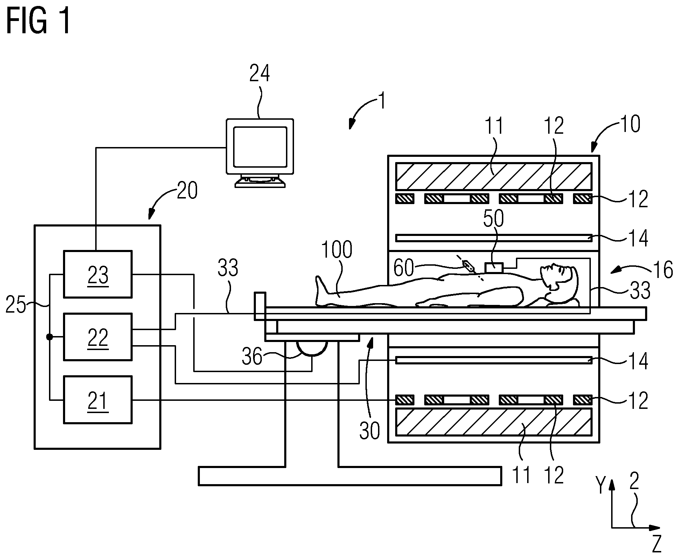

Fig. 1 shows a schematic diagram of a form of embodiment of an inventive magnetic resonance tomography unit 1.

The magnet unit 10 has a field magnet 11, which generates a static magnetic field B0 for alignment of nuclear spins of samples or patients 100 in a recording region. The recording region is arranged in a patient tunnel 16, which extends in a longitudinal direction 2 through the magnet unit 10. A patient 100 may be moved by means of the patient couch 30 and the drive unit 36 of the patient couch 30 into the recording region. Usually, the field magnet 11 involves a superconducting magnet, which can provide magnetic fields with a magnetic flux density of up to 3T, with the very latest devices of even more. For lower field strengths however permanent magnets or electromagnets with normally conducting coils can be employed.

The magnet unit 10 furthermore has gradient coils 12, which are designed, for spatial differentiation of the recorded imaging regions in the examination volume, to superimpose variable magnetic fields in three spatial directions on the magnetic field B0. The gradient coils 12 are usually coils made of normally conducting wires, which can generate fields orthogonal to each other in the examination volume.

The magnet unit 10 likewise has a body coil 14, which is designed to radiate a radio-frequency signal supplied via a signal line into the examination volume and to receive resonant signals emitted by the patient 100 and output them via a signal line 33. In a preferable manner however the body coil 14 is replaced, for sending out the radio-frequency signals and/or receiving them, by the local coil 50, which is arranged in the patient tunnel 16 close to the patient 100. It is also conceivable however for the local coil 50 to be designed for sending and receiving and therefore a body coil 14 can be omitted.

A control unit 20 supplies the magnet unit 10 with the various signals for the gradient coils 12 and the body coil 14 and evaluates the received signals. A magnetic resonance tomography unit controller 23 coordinates the subunits in this case.

Thus the control unit 20 has a gradient control 21, which is designed to supply the gradient coils 12 via supply leads with variable currents, which provide the desired gradient fields coordinated in time in the examination volume.

The control unit 20 furthermore has radio-frequency unit 22, which is designed to generate a radio-frequency pulse with a predetermined timing curve, amplitude and spectral power distribution to excite a magnetic resonance of the nuclear spins in the patient 100. In this case pulse powers in the range of kilowatts can be achieved. The individual units are linked to one another via a signal bus 25.

The radio-frequency signal generated by the radio-frequency unit 22 is conveyed via a signal connection to the body coil 14 and emitted into the body of the patient 100, in order to excite the nuclear spins there. Also conceivable is however an emission of the radio-frequency signal via one or more coil windings of the local coil 50.

The local coil 50 then preferably receives a magnetic resonance signal from the body of the patient 100 since, because of the short distance, the signal-to-noise ratio (SNR) of the local coils 50 is better than a receipt by the body coil 14. The MR signal received by the local coil 50 is preprocessed in the local coil 50 and forwarded to the radio-frequency unit 22 of the magnetic resonance tomography unit 1 for evaluation and image acquisition.

The signal connection between local coil 50 and magnetic resonance tomography unit 1 may be via a signal line 33 or wireless.

The medical instrument 60 may be guided by an operator using the imaging capabilities of the magnetic resonance tomography unit 1 with the method according to the invention as discussed below.

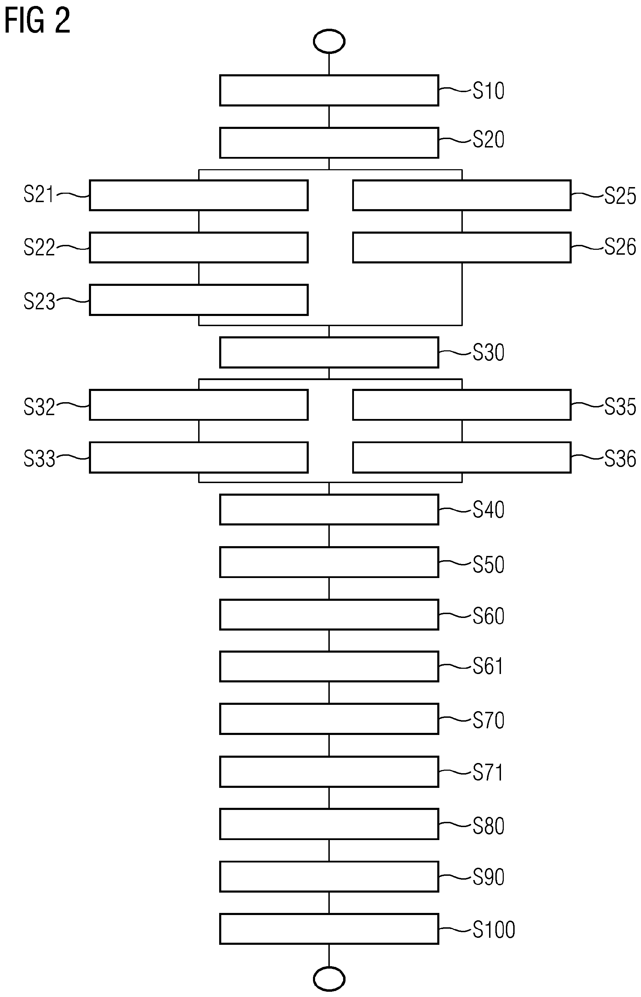

Fig. 2 shows a flow chart of an embodiment of the method for tracking a medical instrument according to the invention.

The method is executed by a magnetic resonance tomography unit comprising 1 a control unit 20 which is configured to perform the steps of an image acquisition with the magnetic resonance tomography unit 1 and in particular the steps of the method according to the invention.

In a step S10 a tracking volume 70 is defined by the control unit 20, which encloses a predefined trajectory 71. The trajectory 71 may e.g. be derived from a 3D model of the patient 100 by an operator. The trajectory 71 may be a linear path between an entrance point and a target destination in the body of a patient 100. The trajectory 71 may also be curved, e.g., in case the medical instrument is a catheter, and it is delimited by a vessel of a patient. A tracking volume 70 may e.g. be defined by a cuboid with the coordinates of the corners given by the minima and maxima of the x, y, z- coordinates of all points along the trajectory 71.

In a step S20, a first 2D projection image of the tracking volume in presence of the medical instrument is acquired by the control unit 20 using the magnetic resonance tomography unit 1. A 2D projection image is an image, which depicts a 3D volume by an 2D image in a plane or piece of a plane by a projection of volume elements along a projection vector or projection direction onto the plane. Two exemplary different ways of acquiring such 2D image projections will be explained in the following paragraphs.

According to the invention, a 2D projection image is acquired by first providing 1D projections of multiple slices of a volume and combining then these 1D projections to a 2D projection image. In a step S21, the tracking volume 70 is therefore divided into a stack of slices 72 by the control unit 20. A slice 72 is an essentially two-dimensional layer of volume elements to be imaged arranged in preferably a plane. The plane may be distorted or warped by non-linearities of the gradient system. The stack of slices 72 may e.g. be arranged along the longitudinal direction 2 along the z-axis parallel to the B0 magnetic field, wherein the planes of the slices 72 are perpendicular to the z-axis. But any other orientation of the stack is considerable, e.g. along x-axis or y-axis or any linear combination thereof.

Preferably the slices 72 of a stack are of equal thickness and/or the planes are arranged at equal distance and fully cover the tracking volume 70 such that there is no place inside the tracking volume 70 which is not included when acquiring MR data for all volume elements 73 of the slices 72.

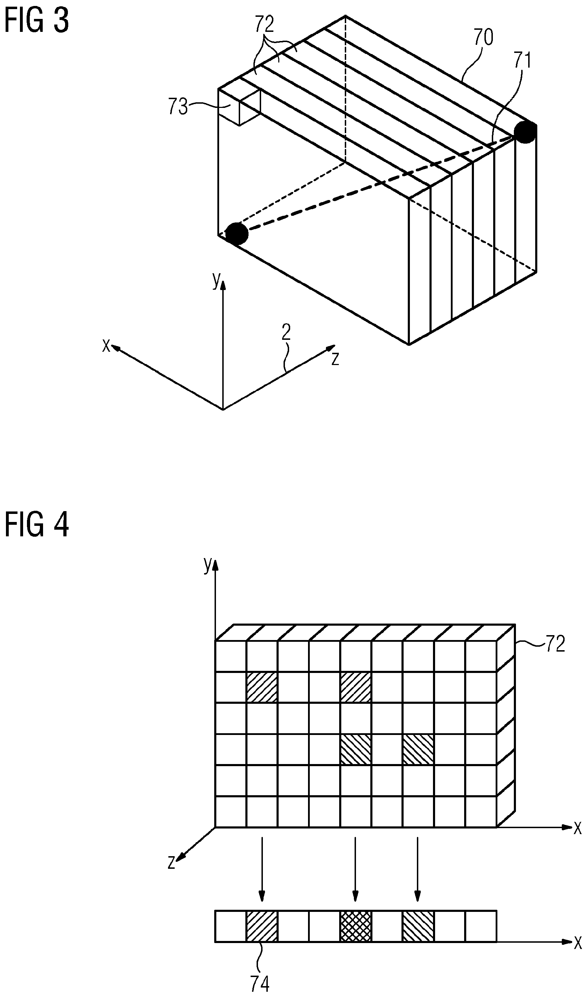

In Fig. 3, such an exemplary stack of slices 72 is shown. For simplicity of the drawing, the slices are arranged in parallel to the x-y-plane of the coordinate system and the stack of slices 72 is arranged along the z-direction. Only a single volume element 73 is shown, but each of the slices is fully covered by a matrix of such volume elements 73.

In another step S22, the control unit 20 acquires a first 1D projection for each slice 72, using the magnetic resonance tomography unit 1.

Such a 1D-projection is shown exemplary in Fig. 4 for a single slice 72. The slice 72 is arranged parallel to the x-y-plane. Such a slice 72 can e.g. be captured by sending an excitation pulse for a narrow frequency band while a z-gradient magnetic field is applied by the control unit 20 using the gradient coils. The position along the z-axis is defined by the center frequency of the pulse while thickness of the slice 72 along the z-axis is defined by the bandwidth of the pulse.

In the example of Fig. 4, only a one-dimensional encoding is applied to the slice 72 such that MR signals with the same x-coordinate experience the same phase shift, independently from the y-coordinate. In the read out, all MR signals from a column with same x-coordinate are summed up. Signals deriving from different y-coordinates are summed up and cannot be separated anymore. This is similar to a simple x-ray image, summing up the attenuation factors of all volume elements along the direction of the x-ray beam, here equal to the y-axis. Thereby, each slice 72 is reduced to an image line 74 of pixels, similar to the lines on an old cathode ray tube.

In another step S23 the stack of first 1D projections is combined by the control unit 20 to a first 2D reference image. Following the example of Fig. 4, the resulting image lines 74 in are arranged in parallel to each other in the x-z-plane such that the pixel elements of each image line 74 in x-direction together with the neighboring pixels of the other slices 72 complement to a two-dimensional image in the x-z-plane. In a beneficial way, the image provides x-z coordinates for any object in the slices 74 above the x-z-plane independent from the y coordinate in the slices 74 by the projection along the y-axis.

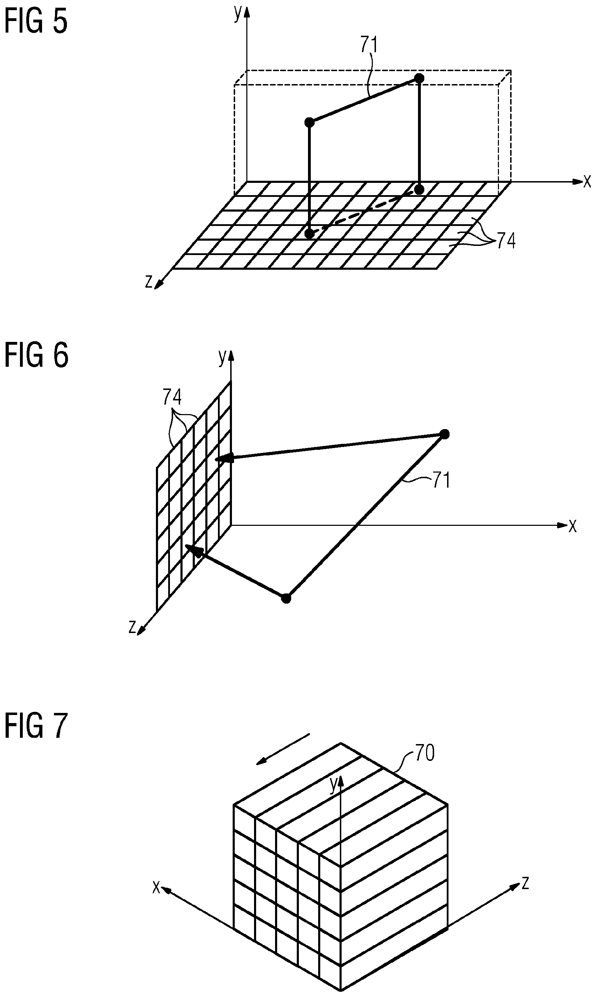

In steps S32 and S33, the steps S22 and S23 are repeated with another coordinate system, e.g. the roles of x and y axis may be swapped such that the slices are projected along the x-axis onto an image line 74 extending along the y-axis, as shown in Fig. 6. This is equivalent to a rotation of the coordinate system around the z-axis by 90 degrees. The idea behind is that the projections in two different directions each provide a pair of two coordinates for each point projected. If the projection directions are not parallel and preferably include an angle larger than 45 degrees or 60 degrees, in particular equal to 90 degrees, the two pairs of two-dimensional coordinates in the projections may be used to determine a three-dimensional coordinate of each point projected.

Alternatively, and outside the scope of the invention as defined by the claims, a 2D projection image may also be acquired with 2D encoding. As shown in Fig. 7, the tracking volume 70 may also be seen as a single slice, which is subdivided in columns in the x-y-axis extending in z-direction through the slice. In a similar way, a single slice could be defined in the y-z-plane with columns extending in x-direction.

For the 2D encoding, all spins to be imaged in the slice have to be excited first by an excitation pulse. This may be achieved in steps S25, S35 by a narrowband pulse at the Larmor frequency in absence of magnetic gradient field. But in this case, also spins outside the tracking volume 70 may be excited and contributed to the MR signal. Alternatively, the excitation may be limited to the thickness of the single slice by applying a broadband excitation pulse, wherein the bandwidth is determined by the applied magnetic gradient in direction of the thickness of the slice and the thickness.

The 2D projection is then acquired by reading out the MR signals for the tracking volume by applying a 2D encoding in Steps S26, S36. The encoding scheme could be e.g. a 2D phase encoding.

A second 2D projection image is acquired in steps S35, S36 wherein at least the step 36 differs from the step S26 by the plane in which the 2D-encoding is provided. The resulting two 2D projection images show the tracking volume from two aspect directions which enclose an angle larger than 30 degrees, 60 degrees, preferably equal to 90 degrees.

In the examples of Figs. 5 and 6, the 2D projection images of Fig. 5 provides x-z-coordinates and Fig. 6 y-z-coordinates such that for an object, which can be identified unambiguously in both projections, the coordinates x, y and z are directly given. For planes at other angles to each other and other orientation to the coordinate system of x, y and z, a coordinate transformation by a matrix may be necessary.

In another step S40, a first reference 2D image is determined by the control unit 20. There are different options how the control unit 20 may perform this step.

A separate 2D projection image may be acquired as explained before in regard to steps S20 and S30, but in absence of the medical instrument, such that the reference 2D image only depicts the patient 100.

It is also conceivable, that the control unit 20 provides a library of reference 2D images by acquiring a series of 2D projection images at different positions of the patient 100. The position is not limited to the body surface of the patient 100, but is in particular related to the organs of the patient 100 in the tracking volume, which may change their position, e.g. as the patient breaths or with the heart beat. The library or atlas of 2D reference images may cover different phases of the heart beat and/or breathing cycle.

It is also conceivable, that the control unit 20 makes use of already acquired 2D projection images for determining a background image. As the position of the medical instrument 60 changes during tracking the instrument, the control unit may filter out constant parts of the 2D projection images to retrieve a background. This could e.g. be achieved by averaging a plurality of images or applying a median function. Also a filter could be applied which reflects the way how the medical instrument is imaged by the magnetic resonance tomography unit 1, e.g. signal extinction.

In a similar way, in another step S50 the control unit 20 determines a second reference 2D image or a library of reference 2D images. The difference is the projection direction or view angle. While the first reference 2D image in step S40 depicts the tracking volume 70 at the same view angle and provides a 2D projection image at the same projection direction than the first 2D projection image, the second 2D reference image depicts the tracking volume 70 at the same view angle than the second 2D reference image.

In another step S60, the control unit 70 suppresses the background from the first 2D projection image as a function of the first reference 2D image. The control unit 70 could e.g. subtract the pixel values of the reference 2D image from the 2D projection image. The reference 2D image could be scaled or distorted before to adjust variations in the image acquisition. The preprocessing could be parametrized and the parameters could be optimized for minimum energy of the resulting differential image.

When a library or atlas of reference 2D images has been acquired for different positions of the patient, the control unit may first select a reference 2D image for background removal from the library which is next to the first 2D projection image. Next may be measured by a metric which e.g. could be provided by a correlation function between 2D projection image and reference 2D image or a minimum energy of a difference image.

The removal of the background, which usually includes the organs of the patient 100 in the tracking volume, simplifies the next step.

In the step S61 a first set of coordinates of the medical instrument from the first 2D image by the control unit.

After the removal of the background, the remaining 2D differential image basically shows the medical instrument 60 or artefacts caused by the medical instrument. A pixel belonging to the medical instrument 60 may be e.g. detected by the fact that the pixel value differs from the average image offset.

The coordinates of the pixels in the image matrix with the signal from the medical instrument 60 define thereby also two coordinates of the medical instrument 60 in the tracking volume. The 2D differential image may also be preprocessed by a filter, e.g. a high pass filter to increase the contrast of the edges of the medical instrument. Also a parametrized model for the medical instrument 60 could be fitted to the 2D differential image to determine the coordinates in a step S61.

In a similar way, the control unit 20 suppresses the background in a step S70 from the second 2D projection image using the second reference 2D image and determines in a step S71 a second set of coordinates from a second 2D differential image retrieved from the second 2D projection image and the second reference 2D image.

In another step S80, from the two sets of 2D coordinates a set of 3D coordinates is determined. For an orthogonal coordinate system and the planes of the first 2D projection image arranged e.g. in the x-y-plane and y-z-plane, the values of the x-y-z coordinates can be derived directly from the coordinates in the planes. For other coordinates, a coordinate transformation may be necessary or solving a linear equation system. It is also conceivable, that the coordinates of a plurality of points of the medical instrument 60 are determined in the same way.

The determined 3D position is output in a step S90 by the control unit 70 to an operator. The position may be depicted on a display in different 2D projections under different view angles. A 3D image could be rendered using the 3D position or a virtual reality showing the medical instrument 60 and the organs in the tracking volume. It is also conceivable to output the 3D coordinates numerically.

In a step S100, the control unit 20 may determine a position of a slice which includes the medical instrument 70, acquire an MR image of the slice and show the image on a display to the operator.

Although the invention has been illustrated and described in greater detail by the preferred exemplary embodiment, the invention is not restricted by the enclosed examples and other variations can be derived herefrom by the person skilled in the art, without departing from the scope of protection of the invention, which is defined by the appended claims.

## Claims

1. Magnetic resonance tomography unit (1) for tracking a medical instrument, wherein the magnetic resonance tomography unit (1) comprises a control unit (20) and wherein the control unit (20) is configured to
- define a tracking volume (70) enclosing a predefined trajectory (71) of the medical instrument in the field of view of the magnetic resonance tomography unit (1);
- acquire a first 2D projection image of the tracking volume (70) in presence of the medical instrument (60) with the magnetic resonance tomography unit (1);
- acquire a second 2D projection image of the tracking volume (70) in presence of the medical instrument (60) with the magnetic resonance tomography unit (1), wherein a first projection vector of the first 2D projection image and a second projection vector of the second 2D projection image enclose an angle larger 20 degrees;
- determine a first reference 2D image;
- determine a second reference 2D image;
- suppress a background from the first 2D projection image as a function of the first reference 2D image;
- determine a first set of coordinates of the medical instrument (60) from the background suppressed first 2D image;
- suppress a background from the second 2D projection image as a function of the second reference 2D image;
- determine a second set of coordinates of the medical instrument from the background suppressed second 2D image;
- determine a 3D position from the first set of coordinates and the second set of coordinates.
**characterized by**, for acquiring the 2D projection images of the tracking volume (70), the control unit (20) being configured to
- divide the tracking volume into a stack of slices (72);
- acquire a first 1D projection for each slice (72) in a first projection direction with the magnetic resonance tomography unit (1);
- combine the stack of first 1D projections to the first 2D projection image;
- acquire a second 1D projection for each slice (72) in a second projection direction with the magnetic resonance tomography unit (1), wherein the first projection direction and the second projection direction enclose an angle larger than 45 degrees, 60 degrees or 80 degrees;
- combine the stack of second 1D projections to the second 2D projection image.

2. Magnetic tomography unit according to claim **1,** wherein the control unit (20) is further configured to output the 3D position of the medical instrument to a user.

3. Magnetic tomography unit according to claim 1 or 2, wherein the control unit (20) is configured to determine the first reference 2D image from a plurality of first 2D projection images and/or to determine the second reference 2D image from a plurality of second 2D projection images.

4. Magnetic tomography unit according to claim 1 or 2, wherein the control unit (20) is configured to determine the first reference 2D image by acquiring a third 2D projection image of the tracking volume (70) in absence of the medical instrument (60) in the tracking volume (70) with the magnetic resonance tomography unit (1) and/or to determine the second reference 2D image by acquiring a fourth 2D projection image of the tracking volume (70) in absence of the medical instrument (60) in the tracking volume (70) with the magnetic resonance tomography unit (1).

5. Magnetic tomography unit according to claim 1, wherein the control unit (20) is further configured to acquire a MR image for a 2D slice comprising the medical instrument (60), wherein the control unit (20) is configured to determine the position of the 2D slice in dependence of the first set of coordinates and/or second set of coordinates.

6. Magnetic tomography unit according to claim 1, wherein the control unit (20) is configured to provide a plurality of the first reference 2D images and/or of the second reference 2D images by repeating the determination of a first reference 2D image and/or determination of a second reference 2D image at different positions of a patient (100), wherein each of the plurality of the first reference 2D images and/or of the second reference 2D images represents a different position of the patient (100).

7. Magnetic tomography unit according to claim 6, wherein the control unit is configured to select for removing the background the first reference 2D image of the plurality of first reference 2D images, which is most similar to the first 2D image and/or the control unit selects the second reference 2D image of the plurality of second reference 2D images, which is most similar to the second 2D image.

8. Magnetic tomography unit according to one of the previous claims, wherein the control unit (20) is configured to select a fat suppressing sequence for acquiring a 2D projection image.

9. Magnetic tomography unit according to one of the previous claims, wherein the control unit (20) is configured to determine a first set of coordinates of the medical instrument (60) from the background suppressed first 2D image and /or (S81) to determine a second set of coordinates of the medical instrument (60) from the background suppressed second 2D image using a peak detection algorithm.

10. Method for tracking a medical instrument (60) with a magnetic resonance tomography unit (1), wherein the magnetic resonance tomography unit (1) comprises a control unit (20) and wherein the method comprises the steps:
(S10) defining a tracking volume (70) enclosing a predefined trajectory (71) of the medical instrument in a field of view of the magnetic resonance tomography unit (1) by the control unit (20);
(S20) acquiring a first 2D projection image of the tracking volume (70) in presence of the medical instrument (60) with the magnetic resonance tomography unit (1);
(S30) acquiring a second 2D projection image of the tracking volume (70) in presence of the medical instrument (60) with the magnetic resonance tomography unit (1), wherein a first projection vector of the first 2D projection image and a second projection vector of the second 2D projection image enclose an angle larger 20 degrees;
(S40) determining a first reference 2D image by the control unit (20);
(S50) determining a second reference 2D image by the control unit (20);
(S60) suppress a background from the first 2D projection image as a function of the first reference 2D image by the control unit (20);
(S61) determining a first set of coordinates of the medical instrument (60) from the background suppressed first 2D image by the control unit (20);
(S70) suppress a background from the second 2D projection image as a function of the second reference 2D image by the control unit (20);
(S71) determining a second set of coordinates of the medical instrument (60) from the background suppressed second 2D image by the control unit 20);
(S80) determining a 3D position from the first set of coordinates and the second set of coordinates by the control unit (20);
**characterized by** the steps of acquiring the 2D projection images (S20, S30) of the tracking volume (70) comprising the steps of
- dividing the tracking volume into a stack of slices (72);
- acquiring a first 1D projection for each slice (72) in a first projection direction with the magnetic resonance tomography unit (1);
- combining the stack of first 1D projections to the first 2D projection image;
- acquiring a second 1D projection for each slice (72) in a second projection direction with the magnetic resonance tomography unit (1), wherein the first projection direction and the second projection direction enclose an angle larger than 45 degrees, 60 degrees or 80 degrees;
- combining the stack of second 1D projections to the second 2D projection image.

11. Method according to claim 10,
wherein the steps (S40) and/or (S50) of determining a 2D reference image are repeated at different positions of a patient (100) wherein each of the plurality of the first reference 2D images and of the second reference 2D images represents a different position of the patient and
wherein during the steps of removing the background (S70, S80) the control unit (20) selects the first reference 2D image of the plurality of first reference 2D images, which is most similar to the first 2D image and/or the control unit (20) selects the second reference 2D image of the plurality of second reference 2D images, which is most similar to the second 2D image.

12. Method according to one of the claims 10 to 11, wherein the medical instrument (60) is a catheter.

13. A computer program product comprising a computer program that is directly loadable into a control unit (20) for a magnetic resonance tomography unit (1) according to one of claims 1 to 9, which comprises program elements for performing the steps of the method according to any of claims 10 to 12 when the computer program is executed by the control unit (20).

## Patentansprüche

1. Magnetresonanztomographieeinheit (1) zum Verfolgen eines medizinischen Instruments, wobei die Magnetresonanztomographieeinheit (1) eine Steuereinheit (20) umfasst und wobei die Steuereinheit (20) dazu ausgelegt ist:
- ein Verfolgungsvolumen (70) zu definieren, das eine vordefinierte Trajektorie (71) des medizinischen Instruments im Sichtfeld der Magnetresonanztomographieeinheit (1) einschließt;
- mit der Magnetresonanztomographieeinheit (1) ein erstes 2D-Projektionsbild des Verfolgungsvolumens (70) in Anwesenheit des medizinischen Instruments (60) zu erfassen;
- mit der Magnetresonanztomographieeinheit (1) ein zweites 2D-Projektionsbild des Verfolgungsvolumens (70) in Anwesenheit des medizinischen Instruments (60) zu erfassen, wobei ein erster Projektionsvektor des ersten 2D-Projektionsbilds und ein zweiter Projektionsvektor des zweiten 2D-Projektionsbilds einen Winkel von mehr als 20 Grad einschließen;
- ein erstes 2D-Referenzbild zu bestimmen;
- ein zweites 2D-Referenzbild zu bestimmen;
- einen Hintergrund aus dem ersten 2D-Projektionsbild als eine Funktion des ersten 2D-Referenzbilds zu unterdrücken;
- aus dem hintergrundunterdrückten ersten 2D-Bild einen ersten Satz von Koordinaten des medizinischen Instruments (60) zu bestimmen;
- einen Hintergrund aus dem zweiten 2D-Projektionsbild als eine Funktion des zweiten 2D-Referenzbilds zu unterdrücken;
- aus dem hintergrundunterdrückten zweiten 2D-Bild einen zweiten Satz von Koordinaten des medizinischen Instruments zu bestimmen;
- aus dem ersten Satz von Koordinaten und dem zweiten Satz von Koordinaten eine 3D-Position zu bestimmen,
**dadurch gekennzeichnet, dass** zum Erfassen der 2D-Projektionsbilder des Verfolgungsvolumens (70) die Steuereinheit (20) dazu ausgelegt ist:
- das Verfolgungsvolumen in einen Stapel von Schichten (72) zu unterteilen;
- mit der Magnetresonanztomographieeinheit (1) eine erste 1D-Projektion für jede Schicht (72) in einer ersten Projektionsrichtung zu erfassen;
- den Stapel erster 1D-Projektionen zu dem ersten 2D-Projektionsbild zu kombinieren;
- mit der Magnetresonanztomographieeinheit (1) eine zweite 1D-Projektion für jede Schicht (72) in einer zweiten Projektionsrichtung zu erfassen, wobei die erste Projektionsrichtung und die zweite Projektionsrichtung einen Winkel von mehr als 45 Grad, 60 Grad oder 80 Grad einschließen;
- den Stapel zweiter 1D-Projektionen zu dem zweiten 2D-Projektionsbild zu kombinieren.

2. Magnettomographieeinheit nach Anspruch 1, wobei die Steuereinheit (20) ferner dazu ausgelegt ist, die 3D-Position des medizinischen Instruments an einen Benutzer auszugeben.

3. Magnettomographieeinheit nach Anspruch 1 oder 2, wobei die Steuereinheit (20) dazu ausgelegt ist, das erste 2D-Referenzbild aus mehreren ersten 2D-Projektionsbildern zu bestimmen und/oder das zweite 2D-Referenzbild aus mehreren zweiten 2D-Projektionsbildern zu bestimmen.

4. Magnettomographieeinheit nach Anspruch 1 oder 2, wobei die Steuereinheit (20) dazu ausgelegt ist, mit der Magnetresonanztomographieeinheit (1) das erste 2D-Referenzbild durch Erfassen eines dritten 2D-Projektionsbilds des Verfolgungsvolumens (70) in Abwesenheit des medizinischen Instruments (60) in dem Verfolgungsvolumen (70) zu bestimmen und/oder mit der Magnetresonanztomographieeinheit (1) das zweite 2D-Referenzbild durch Erfassen eines vierten 2D-Projektionsbilds des Verfolgungsvolumens (70) in Abwesenheit des medizinischen Instruments (60) in dem Verfolgungsvolumen (70) zu bestimmen.

5. Magnettomographieeinheit nach Anspruch 1, wobei die Steuereinheit (20) ferner dazu ausgelegt ist, ein MR-Bild für eine 2D-Schicht, die das medizinische Instrument (60) umfasst, zu erfassen,
wobei die Steuereinheit (20) dazu ausgelegt ist, die Position der 2D-Schicht in Abhängigkeit von dem ersten Satz von Koordinaten und/oder dem zweiten Satz von Koordinaten zu bestimmen.

6. Magnettomographieeinheit nach Anspruch 1, wobei die Steuereinheit (20) dazu ausgelegt ist, mehrere der ersten 2D-Referenzbilder und/oder der zweiten 2D-Referenzbilder bereitzustellen, indem die Bestimmung eines ersten 2D-Referenzbilds und/oder die Bestimmung eines zweiten 2D-Referenzbilds an verschiedenen Positionen eines Patienten (100) wiederholt wird, wobei jedes der mehreren der ersten 2D-Referenzbilder und/oder der zweiten 2D-Referenzbilder eine andere Position des Patienten (100) repräsentiert.

7. Magnettomographieeinheit nach Anspruch 6, wobei die Steuereinheit dazu ausgelegt ist, zum Entfernen des Hintergrunds das erste 2D-Referenzbild der mehreren ersten 2D-Referenzbilder auszuwählen, das dem ersten 2D-Bild am ähnlichsten ist, und/oder die Steuereinheit das zweite 2D-Referenzbild der mehreren zweiten 2D-Referenzbilder auswählt, das dem zweiten 2D-Bild am ähnlichsten ist.

8. Magnettomographieeinheit nach einem der vorhergehenden Ansprüche, wobei die Steuereinheit (20) dazu ausgelegt ist, eine Fettunterdrückungssequenz zum Erfassen eines 2D-Projektionsbilds auszuwählen.

9. Magnettomographieeinheit nach einem der vorhergehenden Ansprüche, wobei die Steuereinheit (20) dazu ausgelegt ist, unter Verwendung eines Peak-Detektionsalgorithmus einen ersten Satz von Koordinaten des medizinischen Instruments (60) aus dem hintergrundunterdrückten ersten 2D-Bild zu bestimmen und/oder (S81) einen zweiten Satz von Koordinaten des medizinischen Instruments (60) aus dem hintergrundunterdrückten zweiten 2D-Bild zu bestimmen.

10. Verfahren zum Verfolgen eines medizinischen Instruments (60) mit einer Magnetresonanztomographieeinheit (1), wobei die Magnetresonanztomographieeinheit (1) eine Steuereinheit (20) umfasst und wobei das Verfahren die folgenden Schritte umfasst:
(S10) Definieren eines Verfolgungsvolumens (70), das eine vordefinierte Trajektorie (71) des medizinischen Instruments in einem Sichtfeld der Magnetresonanztomographieeinheit (1) umschließt, durch die Steuereinheit (20);
(S20) Erfassen eines ersten 2D-Projektionsbilds des Verfolgungsvolumens (70) in Anwesenheit des medizinischen Instruments (60) mit der Magnetresonanztomographieeinheit (1);
(S30) Erfassen eines zweiten 2D-Projektionsbilds des Verfolgungsvolumens (70) in Anwesenheit des medizinischen Instruments (60) mit der Magnetresonanztomographieeinheit (1),
wobei ein erster Projektionsvektor des ersten 2D-Projektionsbilds und ein zweiter Projektionsvektor des zweiten 2D-Projektionsbilds einen Winkel von mehr als 20 Grad einschließen;
(S40) Bestimmen eines ersten 2D-Referenzbilds durch die Steuereinheit (20);
(S50) Bestimmen eines zweiten 2D-Referenzbilds durch die Steuereinheit (20);
(S60) Unterdrücken eines Hintergrunds aus dem ersten 2D-Projektionsbild als eine Funktion des ersten 2D-Referenzbilds durch die Steuereinheit (20);
(S61) Bestimmen eines ersten Satzes von Koordinaten des medizinischen Instruments (60) aus dem hintergrundunterdrückten ersten 2D-Bild durch die Steuereinheit (20);
(S70) Unterdrücken eines Hintergrunds aus dem zweiten 2D-Projektionsbild als eine Funktion des zweiten 2D-Referenzbilds durch die Steuereinheit (20);
(S71) Bestimmen eines zweiten Satzes von Koordinaten des medizinischen Instruments (60) aus dem hintergrundunterdrückten zweiten 2D-Bild durch die Steuereinheit (20);
(S80) Bestimmen einer 3D-Position aus dem ersten Satz von Koordinaten und dem zweiten Satz von Koordinaten durch die Steuereinheit (20);
**gekennzeichnet durch** die Schritte des Erfassens der 2D-Projektionsbilder (S20, S30) des Verfolgungsvolumens (70), die die folgenden Schritte umfassen:
- Unterteilen des Verfolgungsvolumens in einen Stapel von Schichten (72);
- Erfassen einer ersten 1D-Projektion für jede Schicht (72) in einer ersten Projektionsrichtung mit der Magnetresonanztomographieeinheit (1);
- Kombinieren des Stapels erster 1D-Projektionen zu dem ersten 2D-Projektionsbild;
- Erfassen einer zweiten 1D-Projektion für jede Schicht (72) in einer zweiten Projektionsrichtung mit der Magnetresonanztomographieeinheit (1), wobei die erste Projektionsrichtung und die zweite Projektionsrichtung einen Winkel von mehr als 45 Grad, 60 Grad oder 80 Grad einschließen;
- Kombinieren des Stapels zweiter 1D-Projektionen zu dem zweiten 2D-Projektionsbild.

11. Verfahren nach Anspruch 10, wobei die Schritte (S40) und/oder (S50) des Bestimmens eines 2D-Referenzbilds an verschiedenen Positionen eines Patienten (100) wiederholt werden, wobei jedes der mehreren der ersten 2D-Referenzbilder und der zweiten 2D-Referenzbilder eine andere Position des Patienten repräsentiert und
wobei während der Schritte des Entfernens des Hintergrunds (S70, S80) die Steuereinheit (20) das erste 2D-Referenzbild der mehreren ersten 2D-Referenzbilder auswählt, das dem ersten 2D-Bild am ähnlichsten ist, und/oder die Steuereinheit (20) das zweite 2D-Referenzbild der mehreren zweiten 2D-Referenzbilder auswählt, das dem zweiten 2D-Bild am ähnlichsten ist.

12. Verfahren nach einem der Ansprüche 10 bis 11, wobei das medizinische Instrument (60) ein Katheter ist.

13. Computerprogrammprodukt, umfassend ein Computerprogramm, das direkt in eine Steuereinheit (20) für eine Magnetresonanztomographieeinheit (1) nach einem der Ansprüche 1 bis 9 geladen werden kann und das Programmelemente zum Durchführen der Schritte des Verfahrens nach einem der Ansprüche 10 bis 12 bei Ausführung des Computerprogramms durch die Steuereinheit (20) umfasst.

## Revendications

1. Unité (1) de tomodensitométrie par résonnance magnétique pour suivre un instrument médical, dans laquelle l'unité (1) de tomodensitométrie par résonnance magnétique comprend une unité (20) de commande et dans laquelle l'unité (20) de commande est configurée pour
- définir un volume (70) de suivi enfermant une trajectoire (71) définie à l'avance de l'instrument médical dans le champ de vue de l'unité (1) de tomodensitométrie par résonnance magnétique ;
- acquérir, par l'unité (1) de tomodensitométrie par résonnance magnétique, une première image de projection en 2D du volume (70) de suivi en présence de l'instrument (60) médical ;
- acquérir, par l'unité (1) de tomodensitométrie par résonnance magnétique, une deuxième image de projection en 2D du volume (70) de suivi en présence de l'instrument (60) médical, dans laquelle un premier vecteur de projection de la première image de projection en 2D et un deuxième vecteur de projection de la deuxième image de projection en 2D enferment un angle plus grand que 20 degrés ;
- déterminer une première image de référence en 2D ;
- déterminer une deuxième image de référence en 2D ;
- supprimer un arrière-plan de la première image de projection en 2D en fonction de la première image de référence en 2D ;
- déterminer un premier ensemble de coordonnées de l'instrument (60) médical à partir de la première image en 2D avec suppression de l'arrière-plan ;
- supprimer un arrière-plan de la deuxième image de projection en 2D en fonction de la deuxième image de référence en 2D ;
- déterminer un deuxième ensemble de coordonnées de l'instrument médical à partir de la deuxième image en 2D avec suppression de l'arrière-plan ;
- déterminer une position en 3D à partir du premier ensemble de coordonnées et du deuxième ensemble de coordonnées,
**caractérisée par**,
pour acquérir les images de projection en 2D du volume (70) de suivi, l'unité (20) de commande étant configurée pour
- subdiviser le volume de suivi en une pile de tranches (72) ;
- acquérir une première image de projection en 1D pour chaque tranche (72) dans une première direction de projection par l'unité (1) de tomodensitométrie par résonnance magnétique ;
- combiner la pile de premières projections en 1D en la première image de projection en 2D ;
- acquérir une deuxième projection en 1D pour chaque tranche (72) dans une deuxième direction de projection par l'unité (1) d tomodensitométrie par résonnance magnétique, dans laquelle la première direction de projection et la deuxième direction de projection enferment un angle plus grand que 45 degrés, 60 degrés ou 80 degrés ;
- combiner la pile de deuxièmes projections en 1D en la deuxième image de projection en 2D.

2. Unité de tomodensitométrie par résonnance magnétique suivant la revendication 1, dans laquelle l'unité (20) de commande est configurée en outre pour donner la position en 3D de l'instrument médical à un utilisateur.

3. Unité de tomodensitométrie par résonnance magnétique suivant la revendication 1 ou 2, dans laquelle l'unité (20) de commande est configurée pour déterminer la première image de référence en 2D parmi une pluralité de premières images de projection en 2D et/ou pour déterminer la deuxième image de référence en 2D parmi une pluralité de deuxièmes images de projection en 2D.

4. Unité de tomodensitométrie par résonnance magnétique suivant la revendication 1 ou 2, dans laquelle l'unité (20) de commande est configurée pour déterminer la première image de référence en 2D par acquisition, par l'unité (1) de tomodensitométrie par résonnance magnétique, d'une troisième image de projection en 2D du volume (70) de suivi en l'absence de l'instrument (60) médical dans le volume (70) de suivi et/ou pour déterminer la deuxième image de référence en 2D en acquérant, par l'unité (1) de tomodensitométrie par résonnance magnétique, une quatrième image de projection en 2D du volume (70) de suivi en l'absence de l'instrument (60) médical dans le volume (70) de suivi.

5. Unité de tomodensitométrie par résonnance magnétique suivant la revendication 1, dans laquelle l'unité (20) de commande est configurée en outre pour acquérir une image RM pour une tranche en 2D comprenant l'instrument (60) médical, dans laquelle l'unité (20) de commande est configurée pour déterminer la position de la tranche en 2D en fonction du premier ensemble de coordonnées et/ou du deuxième ensemble de coordonnées.

6. Unité de tomodensitométrie par résonnance magnétique suivant la revendication 1, dans laquelle l'unité (20) de commande est configurée pour donner une pluralité des premières images de référence en 2D et/ou des deuxièmes images de référence en 2D, en répétant la détermination d'une première image de référence en 2D et/ou la détermination d'une deuxième image de référence en 2D en des positions différentes d'un patient (100), dans laquelle chacune de la pluralité des premières images de référence en 2D et/ou de la pluralité des deuxièmes images de référence en 2D représente une position différente du patient (100).

7. Unité de tomodensitométrie par résonnance magnétique suivant la revendication 6, dans laquelle l'unité de commande est configurée pour sélectionner pour retirer l'arrière-plan de la première image de référence en 2D de la pluralité de premières images de référence en 2D, qui est la plus semblable à la première image en 2D, et/ou l'unité de commande sélectionne la deuxième image de référence en 2D de la pluralité de deuxièmes images de référence en 2D, qui est la plus semblable à la deuxième image en 2D.

8. Unité de tomodensitométrie par résonnance magnétique suivant l'une des revendications précédentes, dans laquelle l'unité (20) de commande est configurée pour sélectionner une séquence de suppression de graisse pour acquérir une image de projection en 2D.

9. Unité de tomodensitométrie par résonnance magnétique suivant l'une des revendications précédentes, dans laquelle l'unité (20) de commande est configurée pour déterminer un premier ensemble de coordonnées de l'instrument (60) médical à partir de la première image en 2D avec suppression de l'arrière-plan et/ou (S81) pour déterminer un deuxième ensemble de coordonnées de l'instrument (60) médical à partir de la deuxième image en 2D avec suppression de l'arrière-plan en utilisant un algorithme de détection de pic.

10. Procédé de suivi d'un instrument (60) médical par une unité (1) de tomodensitométrie par résonnance magnétique, dans lequel l'unité (1) de tomodensitométrie par résonnance magnétique comprend une unité (20) de commande et dans lequel le procédé comprend les stades :
(S10) définir, par l'unité (20) de commande, un volume (70) de suivi enfermant une trajectoire (71) définie à l'avance de l'instrument médical dans un champ de vue de l'unité (1) de tomodensitométrie par résonnance ;
(S20) acquérir, par l'unité (1) de tomodensitométrie par résonnance magnétique, une première image de projection en 2D du volume (70) de suivi en présence de l'instrument (60) médical ;
(S30) acquérir, par l'unité (1) de tomodensitométrie par résonnance magnétique, une deuxième image de projection en 2D du volume (70) de suivi en présence de l'instrument (60) médical, dans lequel un premier vecteur de projection de la première image de projection en 2D et un deuxième vecteur de projection de la deuxième image de projection en 2D enferment un angle plus grand que 20 degrés ;
(S40) déterminer une première image de référence en 2D par l'unité (20) de commande ;
(S50) déterminer une deuxième image de référence en 2D par l'unité (20) de commande ;
(S60) supprimer, par l'unité (20) de commande, un arrière-plan de la première image de projection en 2D en fonction de la première image de référence en 2D ;
(S61) déterminer, par l'unité (20) de commande, un premier ensemble de coordonnées de l'instrument (60) médical à partir de la première image en 2D avec suppression de l'arrière-plan ;
(S70) supprimer, par l'unité (20) de commande, un arrière-plan de la deuxième image de projection en 2D en fonction de la deuxième image de référence en 2D ;
(S71) déterminer, par l'unité (20) de commande, un deuxième ensemble de coordonnées de l'instrument (60) médical à partir de la deuxième image en 2D avec suppression de l'arrière-plan ;
(S80) déterminer, par l'unité (20) de commande, une position en 3D à partir du premier ensemble de coordonnées et du deuxième ensemble de coordonnées ;
**caractérisé par** les stades d'acquisition des images (S20, S30) de projection en 2D du volume (70) de suivi comprenant les stades de :
- subdiviser le volume de suivi en une pile de tranches (72) ;
- acquérir, par l'unité (1) de tomodensitométrie par résonnance magnétique, une première image de projection en 1D pour chaque tranche (72) dans une première direction de projection ;
- combiner la pile de premières projections en 1D en la première image de projection en 2D ;
- acquérir, par l'unité (1) d tomodensitométrie par résonnance magnétique, une deuxième projection en 1D pour chaque tranche (72) dans une deuxième direction de projection, dans lequel la première direction de projection et la deuxième direction de projection enferment un angle plus grand que 45 degrés, 60 degrés ou 80 degrés ;
- combiner la pile de deuxièmes projections en 1D en la deuxième image de projection en 2D.

11. Procédé suivant la revendication 10, dans lequel on répète les stades (S40) et/ou (S50) de détermination d'une image de référence en 2D en des positions différentes d'un patient (100), dans lequel chacune de la pluralité des premières images de référence en 2D et/ou des deuxièmes images de référence en 2D représente une position différente du patient, et
dans lequel, pendant les stades de retrait de l'arrière-plan (S70, S80), l'unité (20) de commande sélectionne la première image de référence en 2D de la pluralité de premières images de référence en 2D, qui est la plus semblable à la première image en 2D, et/ou l'unité (20) de commande sélectionne la deuxième image de référence en 2D de la pluralité de deuxièmes images de référence en 2D, qui est la plus semblable à la deuxième image en 2D.

12. Procédé suivant l'une des revendications 10 à 11, dans lequel l'instrument (60) médical est un cathéter.

13. Un produit de programme d'ordinateur comprenant un programme d'ordinateur, qui peut être chargé directement dans une unité (20) de commande d'une unité (1) de tomodensitométrie par résonnance magnétique suivant l'une des revendications 1 à 9, qui comprend les éléments de programme pour effectuer les stades du procédé suivant l'une quelconque des revendications 10 à 12, lorsque le programme d'ordinateur est exécuté par l'unité (20) de commande.
